# EUROPEAN PATENT SPECIFICATION

(11) **EP 0 423 436 B1**
(45) Date of publication and mention of the grant of the patent: **15.02.1995**
(21) Application number: 90113965.9
(22) Date of filing: 20.07.1990
(51) Int. Cl.: A61F 13/15

(54) **Liquid absorbing pad and method of making same**
Flüssigkeitsabsorbierendes Kissen und Verfahren zu seiner Herstellung
Tampon absorbant de liquide et son procédé de fabrication

(30) Priority: 20.10.1989 US 424538
(43) Date of publication of application: 24.04.1991
(73) Proprietor: Standard Textile Company, Inc, Cincinnati Ohio 45237 (US)
(72) Inventor: Heiman, Mark J., Maineville, Ohio 45039 (US)
(74) Representative: Klunker . Schmitt-Nilson . Hirsch

(56) References cited:
- WO-A-86/05386
- AU-A- 509 028
- DE-B- 1 073 149
- FR-A- 1 413 802
- GB-A- 2 198 355

## Description

This invention relates to a liquid-absorbing pad and, in particular, to a pad according to the preamble of claim 1.

It is known in the art to provide a liquid-absorbing pad which comprises a liquid impervious layer having opposed surfaces and absorbing means attached to the layer against one of the opposed surfaces and such liquid-absorbing pad has been proposed for use as a bed pad, as a chair pad, and as a diaper.

GB-A-2 198 355, for example, describes an incontinence pad that includes a sheet of foamed plastics material, a sheet of single sided terry fabric overlying the plastics material with the pile facing away from the latter, the plastics material being bonded to the terry fabric.

WO-A-8 605 386 describes an incontinence pad that includes a layer of permeable hydrophobic material, for example, polyester-viscous material that is secured to an absorbent pad of toweling material so that, in use, urine passes through the hydrophobic material into the absorbent pad to increase the comfort of the wearer. However, the layer of hydrophobic material is a separate structural layer that is separately secured to the toweling material.

One feature of this invention is to provided a new liquid-absorbing pad. The term "liquid-absorbing" is used throughout this specification to describe the pad of this invention and means a pad that provides a "wicking" action and an adsorption and absorption of liquid as more fully described at the end of this specification. The new liquid-absorbing pad of this invention is particularly adapted to be used with a patient having urinary incontinence. The pad is also effective in absorbig other liquids as well, such as, blood in the case of a surgical patient; and, the pad assembly serves to keep liquids away from a patient's body so as to assure optimum comfort and the prevention of decubitis ulcers and the like. The pad may also be used to make both adult and infant diapers as well as other products where it is desired to have a liquid-absorbing means cooperating with a liquid impervious layer for any purpose.

The new liquid absorbent incontinent pad of this invention is characterized by the features as disclosed in the characterizing clause of the attached claim 1.

Other features, objects, uses, and advantages of this invention are apparent from a reading of this description which proceed with reference to the accompanying drawings forming a part thereof.

### BRIEF DESCRIPTION OF THE DRAWINGS

The accompanying drawings show present preferred embodiment of this invention, in which
Fig. 1 is a plan view of one exemplary embodiment of the liquid-absorbing pad of this invention with the central portion thereof broken away;
Fig 2. is an enlarged cross-sectional view taken essentially on the line 2-2 of Fig. 1;
Fig 3. is a schematic cross-sectional view particularly illustrating the three component portions comprising the integral single layer which defines the liquid-absorbing means of the pad of Fig. 1; and
Fig. 4 is a fragmentary isometric view presented to show the overall stacked arrangement of the liquid impervious layer and its components and the single layer of knitted cloth which defines the two-layer construction of the liquid-absorbing pad of this invention.

### DESCRIPTION OF THE PREFERRED EMBODIMENTS

While the various features of this invention are hereinafter illustrated and described as being particularly adapted to provide a liquid-absorbing pad usable as a bed pad, chair pad, wheelchair pad, diaper, and the like, in a manner known in the art, it is to be understood that the various features of this invention can be utilized singly or in various combinations thereof to provide a liquid-absorbing pad usable with other liquids and in other applications, as desired.

Therefore, this invention is not to be limited to only the embodiments illustrated in the drawings because the drawings are merely utilized to illustrate exemplary ones of the wide variety of uses of this invention.

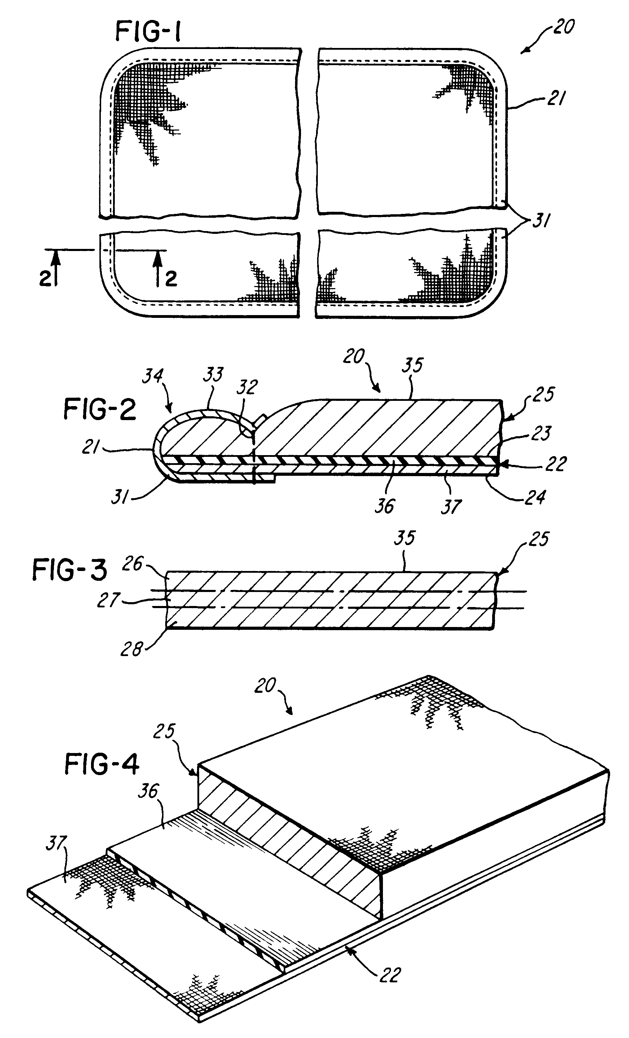

Reference is now made to Fig. 1 of the drawings which illustrates one exemplary embodiment of the pad of this invention which is designed generally by the reference numeral 20. The pad 20 is particularly adapted to be used as a bed pad, chair pad, wheelchair pad, diaper, or the like; and, such pad is particularly adapted for use with a patient having urinary incontinence, or anyone subject to the drainage of body fluids.

As seen in Figs. 1 and 2 of the drawings, the pad has a peripheral outline 21 and comprises a liquid impervious layer designed generally by the reference numeral 22 with the liquid impervious layer having opposed surfaces consisting of an inside surface 23 and an outside surface 24. The pad 20 has absorbing means 25 attached to the layer 22 against one of the opposed surfaces and, in particular, against the inside surface 23.

In accordance with the teachings of this invention, the liquid-absorbing means or layer 25 consists of a single layer of knitted cloth and, as shown schematically in Fig. 3 of the drawings, such single layer of knitted cloth has an exposed front or top portion 26, a central ground or base portion 27, and a back portion 28. In one example of the invention, the single layer 25 was a form of knitted terry cloth. As is known in the knitted terry cloth art, the central base portion serves to hold the front and back portions 26 and 28 respectively together while providing an integral structural separation therebetween.

Referring again to Fig. 1 of the drawings, the pad 20 preferably has rounded corners and also preferably has tape 31 which protects the peripheral edges of its layers 22 and 25, holds layers 22 and 25 together, and defines the peripheral outline 21 of such pad. The tape 31 may be any suitable tape known in the art and may be made of any suitable material. Preferably the tape 31 is made of 100% polyester and is in the form of a so-called bias binding tape which is fastened in position by suitable stitch means or stitches 32 as shown in Fig. 2 of the drawings. The techniques used to define the rounded corners of the pad 20 and the manner of attaching the tape 31 at such corners is well known in the art. Once the tape 31 is stitched in position, the pad 20 has a compressed peripheral portion defined by an outer portion 33 which has an arcuate configuration as shown at 34. It will also be appreciated that the 100% polyester binding tape 31 can be a twilled or twill tape or may be made of any other suitable material and made by any other suitable weaving process.

The layers 22 and 25 may also be held together by so-called overcast stitching, or the like.

The single layer of knitted cloth 25 may be made entirely of a particular material and in one exemplary embodiment of this invention such particular material is in the form of a polyester. The polyester consists of a single variety of polyester or may consist of a plurality of varieties of polyester as will be described subsequently.

The single layer of knitted cloth may also be made of a plurality of materials and the plurality of materials may consist of various combinations of polyester, cotton, rayon, nylon, and the like. Preferably such plurality of materials consists of two materials; and, in another exemplary embodiment of this invention, the plurality of materials consists of two materials in the form of polyester and cotton. In such other embodiment, the front portion 26 of such single layer is made of 100% polyester and the back portion 28 is made of 100% cotton.

In a single layer 25 made of polyester and cotton and when also considering the central base 27 of the single layer 25, the entire single layer may comprise between 25% and 62% cotton and between 75% and 38% polyester respectively.

The front or top portion 26 of the single layer of terry cloth 25 is preferably made entirely (i.e. 100%) of polyester and this is applicable not only to the layer 25 made entirely of polyester but also to layer 25 made of a plurality of materials. In addition, the ground or central base portion 27 may also be made entirely (i.e. 100%) of polyester or of a combination of polyester and cotton or of 100% cotton. The combination of polyester and cotton of portion 27 may be a blend of 50% cotton and 50% polyester.

However, regardless of whether the single layer 25 is made entirely of a single material such as polyester or a plurality of materials such as polyester and cotton, the weight of the overall layer 25 may range from roughly 7 to 14 ounces per square yard (237 to 475 grams per square meter) with the preferred range being between 8.5 ounces and 12.5 ounces per square yard (288 and 423 grams per square meter).

It will also be appreciated that the single layer 25\may be made such that the front portion 26 is made of 100% polyester and the back portion is made of a blend of polyester and cotton. It will be appreciated that in accordance with techniques known in the art, the quantity of fibers making up such blend of polyester and cotton may vary; however, in one embodiment of this invention a preferred blend of 50% polyester and 50% cotton is employed.

Another characteristic of the improved pad of this invention is that the single layer of knitted cloth is made such that the front portion 26 thereof consists entirely of uncut loops of material which provide a wicking action from the outermost surface 35 of the top portion 26 inwardly toward the back portion 28 of the layer 25. The uncut loops of material are preferably brushed loops further improving the wicking action which is normally provided by the uncut loops of the cloth defining such front portion 26.

The back portion 28 of the single layer 25 is made of material which has a greater capacity to retain liquids as contrasted to the construction of the material defining the front portion 26 which provides the improved wicking action. While the material defining the back portion 28 may be the same basic material as the material of the front portion 26 which has been suitably modified to provide greater absorbency, in one preferred embodiment of this invention, such material is looped, uncut, and unbrushed cotton.

Having described the construction of the pad 20, the detailed description will now proceed with a description of more detailed of the two component layers 22 and 25 which define the pad 20. In particular, the liquid impervious layer or sheet 22 is preferably a sheet capable of withstanding hydrostatic water pressure of 100 pounds per square inch gauge (psig) or 7 kg/cm² without allowing passage of water therethrough. However, it will be appreciated that the particular layer 22 may be constructed so that it can withstand any desired hydrostatic water pressure which may be more or less than 100 psig, as desired.

The layer or sheet 22 is preferably comprised of a polymeric sheet portion 36 and may be comprised of either a knitted or a woven fabric 37 in which the polymeric sheet portion is laminated or coated onto the fabric 37. This may be achieved by extruding a polymeric material directly against the fabric 37 suitably forming the polymeric sheet portion 36 using suitable calendar rolls, or the like, and as is well known in the art. The polymeric sheet portion 36 may also be suitably coated onto the fabric 37 by any suitable means known in the art. The polymeric sheet portion 36 may also be laminated onto the fabric 37 using additional adhesive means, or the like, therebetween. The layer or sheet 22 is available from a number of manufacturers.

The single layer 25 is capable of being produced by various manufacturers, however, one manufacturer is Guilford Mills of Greensboro, North Carolina. A typical manufacturer of layer 25 will have equipment (which is known in the art) capable of producing knitted terry cloth.

It will be appreciated that the various materials selected to define the layers 22 and 25 should be compatible with the liquids which are to be absorbed by the pad 20. In applications where the pad 20 is for a person having urinary incontinence or for the purpose of absorbing urine, the constituents of the pad are such that urine will not cause degradation or damage to the layers 22 and 25. The same principle applies where the pad is particularly adapted to be used with other liquids. It will also be appreciated that the thread defining the stitches or stitch means 32 may be any suitable thread compatible with the liquids to be absorbed by the pad 20 and preferably such thread is made of polyester for a pad likely to wick or absorb urine.

The single layer of knitted cloth and, in particular, the front or top portion 26 of such layer may have a hydrophilic finish and may also have an antimicrobial finish, if desired. The antimicrobial finish, if used, assures that the number of microorganisms will be reduced upon continued contact with such finish.

If desired, the pad 20 may have a hydrophilic finish used thereon. If used, any suitable hydrophilic finish known in the art may be used; and, an exemplary finish which may be used is sold under the trade name "LR Finish" by Lubach International Consultants of Charlotte, North Carolina.

Similarly, any suitable nonleaching antimicrobial finish known in the art may be used. The antimicrobial finish should be compatible with the hydrophilic finish. The antimicrobial finish is useful in killing a wide variety of bacteria, controls fermentation of urine, controls production of ammonia, and controls the production of odor.

As previously mentioned, the layer 25 is in the form of a single layer of knitted cloth such as terry cloth. This is in contrast to woven terry cloth and the knitted terry cloth may be made on various machines known in the textile industry utilizing a plurality of bars. The technique or process employed to make the knitted single layer terry cloth is not a part of this invention nor is the machine employed to make such cloth a part of this invention inasmuch as any suitable technique or machine known in the textile art may be employed, as indicated previously.

It will also be appreciated that, as indicated earlier, various materials and various percentages of such materials may comprise the layer 25 which comprises the pad 20 of this invention. Materials and percentages for such layer may be as shown in the following chart. As indicated previously, the overall weight of the layer 25 may range from 7 to 14 ounces per square yard (237 10475 grams per square meter) with preferred weights being shown on such chart.

| **MATERIALS AND PERCENTAGES FOR LAYER 25 OF PAD 20** | | |
|---|---|---|
| **CONTENT** | **OZ./YD.²** (gms/m²) | **FIBER CONTENT OF ENTIRE FABRIC** |
| 100% Polyester Front -100% Cotton Back | 12.5 (424) | 54 Cotton/46 Polyester |
| 100% Polyester Front -100% Cotton Back | 10.5 (356) | 54 Cotton/46 Polyester |
| 100% Polyester Front -100% Cotton Back | 8.5 (288) | 51 Cotton/49 Polyester |
| 100% Polyester Front -50% Polyester/50% Cotton Back | 12.5 (424) | 25 Cotton/75 Polyester |
| 100% Polyester Front -50% Polyester/50% Cotton Back | 10.5 (356) | 25 Cotton/75 Polyester |
| 100% Polyester Front -50% Polyester/50% Cotton Back | 8.5 (288) | 25 Cotton/75 Polyester |
| 100% Polyester Front -"Great Feelings" Polyester Back | 12.5 (424) | 53 "Great Feelings" Polyester/47 Polyester |
| 100% Polyester Front -"Great Feelings" Polyester Back | 10.5 (356) | 53 "Great Feelings" Polyester/47 Polyester |
| 100% Polyester Front -"Great Feelings" Polyester Back | 8.5 (288) | 53 "Great Feelings Polyester/47 Polyester |

It will also be noted that reference is made to a particular variety of polyester referred to as a so-called "Great Feelings" polyester and such "Great Feelings" polyester is a special polyester which is available from the E.I. Dupont de Nemours and Company of Wilmington, Delaware, and sold under the trade designation "Great Feelings." "Great Feelings" polyester consists of a 50/50 blend of 1.2 denier per filament and 2.0 denier per filament for an average of approximately 1.5 denier per filament. Great Feelings polyester is specifically designed for use in knit structures; and, it contains an added whitener which gives it a blue-white appearance. Great Feelings polyester differs from regular polyester in its blend of denier per filaments in the same yarn whereby more spaces are created within such yarn resulting in an improved capacity to retain liquid.

Thus, it is seen from the above description that this invention provides a new liquid-absorbing pad which provides optimum comfort and hygiene to a person coming into contact therewith. In addition, this invention provides a new method of making a liquid-absorbing pad.

The pad 20 of this invention is such that once liquid is introduced on the top surface or top portion 26 it passes through the central ground or base portion 27 and is retained in the back portion 28; and, such liquid does not pass back through or return from the back portion 28 to the top portion 26.

As mentioned earlier in this specification, the term "liquid-absorbing" as used to define the new pad of this invention has a meaning now to be further explained. In particular, in the pad 20 liquid comes in contact with the top synthetic surface (i.e. top portion 26) of the fabric, it is temporarily held in the interstices of the synthetic yarns by adsorption because the liquid does not actually absorb into the fibers themselves, it wicks along the fibers. Gravity continues to pull the liquid down until it comes in contact with the intermediate layer or base portion 27 and bottom hydrophilic layer or back portion 28. As liquid comes in contact with these two layers 27-28, it begins to be dispersed into the yarns either by adsorption or absorption. It is held in this reservoir pulling it away from the top surface, therefore, leaving the top surface dry in a very short amount of time. It remains in the absorbent layer "bottom" (i.e. back portion 28) until it is removed by laundering.

While the forms of this invention now preferred have been illustrated and described, it is to be understood that other forms can be utilized and still fall within the scope of the appended claims,

## Claims

1. A liquid absorbent incontinent pad (20) adapted to prevent the spread of urine discharged from an incontinent,
said incontinent pad (20) comprising
a first layer (25) adapted to contact the person of an incontinent and absorb liquid discharged by the incontinent,
a second liquid impervious layer (22) forming the lower exterior surface of said pad (20) and
means for securing said frist and second layers (25, 22) in superposed, assembled relation, **characterized in that**
the frist layer (25) is a single layer of knitted double-sided terry cloth having separate liquid wicking (hydrophobic) and liquid retentive (hydrophilic) properties, and comprising
a central ground or base portion (27),
an exposed top portion (26) formed by yarn loops projecting upwardly from the central ground portion and defining a upper surface (35) of said pad (20), said top portion (26) being made entirely of synthetic material, and a back portion (28) formed by hydrophilic yarn loops projecting downwardly from said central ground portion (27) and defining a lower surface of the frist layer (25),
said central ground portion (27) holding the top and back portions (26, 28) together while providing a integral structural separation therebetween, further **characterized in that**
the top portion (26) has the capability of wicking liquid from the upper surface (35) of said pad (20),
the back portion (28) has the capability of absorbing liquid and providing a reservoir function to minimize the liquid in the top portion (26) and particularly the upper surface (35) which is contacted by the incontinent, and
said central ground portion (27) has a construction and liquid retention ability which facilitates dispersal of liquid from the top portion (26) into the back portion (28),
to thereby prevent liquid passing return from the back portion (28) to the top portion (26) so that
the upper surface (35) of the incontinent pad (20) has a dry feel shortly after liquid is discharged thereon.

2. A pad as set forth in claim 1 characterized in that said central base portion (27) is made entirely of polyester.

3. A pad as set forth in claim 1 characterized in that said central base portion (27) is made of a combination of polyester and cotton.

4. A pad as set forth in claim 1 characterized in that said top and said back portions (26, 28) are made entirely of polyester.

5. A pad as set forth in claim 1 characterized in that said central base portion (27) and said back portion (28) of said single layer (25) are made of cotton.

6. A pad as set forth in any of the claims 1 to 5 characterized in that the pad (20) ranges in weight from roughly 8.5 ounces per square yard to roughly 12.5 ounces per square yard (288 to 423 grams per square meter).

7. A pad as set forth in claim 4 characterized in that said polyester of said top portion (26) is a variety of polyester different from the polyester of said central base portion (27) and of said back portion (28).

8. A pad as set forth in any of the claims 1 to 7 characterized in that said single layer (25) ranges in weight from roughly (8.5 ounces per square yard to roughly 12.5 ounces per square yard) 288 to 423 grams per square meter.

9. A pad as set forth in claim 5 characterized in that said back portion (28) of said single layer (25) is made of 100% cotton.

10. A pad as set forth in claim 9 characterized in that said single layer (25), when also considering said central base portion (27), comprises between 25% and 62% cotton and between 75% and 38% polyester respectively.

11. A pad as set forth in claim 10 characterized in that said single layer (25) ranges in weight from roughly (8.5 ounces per square yard to roughly 12.5 ounces per square yard) 288 to 423 grams per square meter.

12. A pad as set forth in claim 1 characterized in that said back portion (28) of said single layer (25) is made of a blend of polyester and cotton.

13. A pad as set forth in claim 12 characterized in that said blend of polyester and cotton in said back portion (28) consists of a blend of 50% polyester and 50% cotton.

14. A pad as set forth in any of the claims 1 to 13 characterized in that said top portion (26) consists entirely of uncut loops of material which provide a wicking action from the outermost surface of said top portion (26).

15. A pad as set forth in claim 14 characterized in that said uncut loops are brushed loops thereby further improving said wicking action.

16. A pad as set forth in claim 1 characterized in that said central base portion (27) is made of a blend of 50% cotton and 50% polyester.

17. A pad as set forth in any of the claims 1 to 16 characterized in that said base portion (27) consists entirely of uncut and unbrushed loops of material.

18. A pad as set forth in claim 1 characterized in that said central base portion (27) and said back portion (28) of said single layer (25) are comprised of a material or blend of materials selected from the group consisting of polyester, cotton, rayon, and nylon.

## Patentansprüche

1. Flüssigkeitsabsorbierendes Inkontinenzkissen (20) zum Verhindern der Ausbreitung von inkontinenzbedingtem Urin, wobei das Inkontinenzkissen (20) aufweist
eine erste Schicht (25) zum Berühren der Person mit der Inkontinenz und zum Absorbieren von durch die Inkontinenz freigesetzter Flüssigkeit,
eine zweite flüssigkeitsundurchlässige Schicht (22), die die untere Außenfläche des Kissens (20) bildet, und
eine Einrichtung zum Halten der ersten und der zweiten Schicht (25, 22) in übereinanderliegender, zusammengefügter Lage,
**dadurch gekennzeichnet**, daß
die erste Schicht (25) eine Einzelschicht aus gestricktem doppelseitigem Frottierstoff mit getrennten flüssigkeitsabweisenden (hydrophoben) und flüssigkeitshaltenden (hydrophilen) Eigenschaften ist und umfaßt:
einen zentralen Grund- oder Basisabschnitt (27),
einen freiliegenden oberen Abschnitt (26), gebildet durch Garnschleifen, die von dem zentralen Grundabschnitt nach oben vorstehen und eine Oberseite (35) des Kissens (20) definieren; wobei der obere Abschnitt (26) vollständig aus Synthetikmaterial hergestellt ist, und
einen hinteren Abschnitt (28), gebildet durch hydrophile Garnschleifen, die von dem zentralen Grundabschnitt (27) nach unten abstehen und eine Unterseite der ersten Schicht (25) definieren,
wobei der zentrale Grundabschnitt (27) den oberen und den hinteren Abschnitt (26, 28) zusammenhält und zwischen ihnen dabei eine integrale bauliche Trennung darstellt, und weiterhin
**dadurch gekennzeichnet**, daß
der obere Abschnitt (26) die Fähigkeit besitzt, Flüssigkeit von der Oberseite (35) des Kissens (20) abzuziehen,
der hintere Abschnitt (28) die Fähigkeit besitzt, Flüssigkeit zu absorbieren und eine Reservoir-Funktion zu erfüllen, um die Flüssigkeit in dem oberen Abschnitt (26) und insbesondere der von der Inkontinenz berührten Oberseite (35) zu minimieren, und
wobei der zentrale Grundabschnitt (27) einen solchen Aufbau und eine solche Flüssigkeitshaltefähigkeit besitzt, daß die Ausbreitung von Flüssigkeit von dem oberen Abschnitt (26) in den hinteren Abschnitt (28) erleichtert wird, um dadurch zu verhindern, daß Flüssigkeit von dem hinteren Abschnitt (28) zurück zu dem oberen Abschnitt (26) gelangt, so daß
die Oberseite (35) des Inkontinenzkissens (20) sich bereits kurz nach dem Aufbringen von Flüssigkeit auf sie trocken anfühlt.

2. Kissen nach Anspruch 1,
**dadurch gekennzeichnet**,
daß der zentrale Basisabschnitt (27) vollständig aus Polyester gefertigt ist.

3. Kissen nach Anspruch 1,
**dadurch gekennzeichnet**,
daß der zentrale Basisabschnitt (27) aus einer Kombination aus Polyester und Baumwolle hergestellt ist.

4. Kissen nach Anspruch 1,
**dadurch gekennzeichnet**,
daß der obere und der hintere Abschnitt (26, 28) vollständig aus Polyester gefertigt sind.

5. Kissen nach Anspruch 1,
**dadurch gekennzeichnet**,
daß der zentrale Basisabschnitt (27) und der hintere Abschnitt (28) der Einzelschicht (25) aus Baumwolle gefertigt sind.

6. Kissen nach irgendeinem der Ansprüche 1 bis 5,
**dadurch gekennzeichnet**,
daß das Gewicht des Kissens (20) von etwa 8,5 Unzen pro Quadrat-Yard bis etwa 12 Unzen pro Quadrat-Yard (288 bis 423g pro Quadratmeter) reicht.

7. Kissen nach Anspruch 4,
**dadurch gekennzeichnet**,
daß das Polyester des oberen Abschnitts (26) eine Variante von Polyester ist, die anders ist als das Polyester des zentralen Basisabschnitts (27) und des hinteren Abschnitts (28).

8. Kissen nach irgendeinem der Ansprüche 1 bis 7,
**dadurch gekennzeichnet**,
daß das Gewicht der Einzelschicht (25) von etwa (8,5 Unzen pro Quadrat-Yard bis etwa 12,5 Unzen pro Quadrat-Yard) 288 bis 423 g pro Quadratmeter reicht.

9. Kissen nach Anspruch 5,
**dadurch gekennzeichnet**,
daß der hintere Abschnitt (28) der Einzelschicht (25) aus 100 % Baumwolle besteht.

10. Kissen nach Anspruch 9,
**dadurch gekennzeichnet**,
daß die Einzelschicht (25) bei Einbeziehung des zentralen Basisabschnitts (27) zwischen 25 % und 62 % Baumwolle und zwischen 45 % und 38 % Polyester aufweist.

11. Kissen nach Anspruch 10,
**dadurch gekennzeichnet**,
daß das Gewicht der Einzelschicht (25) von etwa (8,5 Unzen pro Quadrat-Yard bis etwa 12,5 Unzen pro Quadrat-Yard) 288 bis 423 g pro Quadratmeter reicht.

12. Kissen nach Anspruch 1,
**dadurch gekennzeichnet**,
daß der hintere Abschnitt (20) der Einzelschicht (25) aus einem Gemisch aus Polyester und Baumwolle besteht.

13. Kissen nach Anspruch 12,
**dadurch gekennzeichnet**,
daß das Gemisch aus Polyester und Baumwolle in dem hinteren Abschnitt (28) aus einem Gemisch aus 50 % Polyester und 50 % Baumwolle besteht.

14. Kissen nach irgendeinem der Ansprüche 1 bis 13,
**dadurch gekennzeichnet**,
daß der obere Abschnitt (26) vollständig aus ungeschnittenen Schleifen eines Materials besteht, die eine Dochtwirkung von der äußersten Oberfläche des oberen Abschnitts (26) entfalten.

15. Kissen nach Anspruch 14,
**dadurch gekennzeichnet**,
daß die ungeschnittenen Schleifen gebürstete Schleifen sind, um dadurch die Dochtwirkung weiter zu steigern.

16. Kissen nach Anspruch 1,
**dadurch gekennzeichnet**,
daß der zentrale Basisabschnitt (27) aus einem Gemisch aus 50 % Baumwolle und 50 % Polyester besteht.

17. Kissen nach irgendeinem der Ansprüche 1 bis 16,
**dadurch gekennzeichnet**,
daß der Basisabschnitt (27) vollständig aus ungeschnittenen und ungebürsteten Materialschleifen besteht.

18. Kissen nach Anspruch 1,
**dadurch gekennzeichnet**,
daß der zentrale Basisabschnitt (27) und der hintere Abschnitt (28) der Einzelschicht (25) aus einem Material oder einem Gemisch aus Materialien bestehen, das aus der Gruppe Polyester, Baumwolle, Rayon und Nylon ausgewählt ist.

## Revendications

1. Coussinet absorbant les liquides pour incontinent (20), conçu pour prévenir la dissémination de l'urine excrétée par un incontinent,
ledit coussinet pour incontinent (20) comprenant
une première couche (25) conçue pour être en contact avec l'incontinent et absorber le liquide excrété par l'incontinent,
une seconde couche imperméable aux liquides (22) formant la surface externe inférieure dudit coussinet (20) et
un moyen de fixation desdites première et seconde couches (25, 22) en une relation assemblée superposée, caractérisé en ce que
la première couche (25) est une monocouche de tricot éponge double face ayant des propriétés distinctes de mèche à liquides (hydrophobes) et de rétention de liquides (hydrophiles) et comprenant
une partie fondamentale ou basale centrale (27),
une partie supérieure exposée (26) formée par des boucles de fil faisant saillie vers le haut à partir de la partie basale centrale et définissant une surface supérieure (35) dudit coussinet (20), ladite partie supérieure (26) étant entièrement en matière synthétique et
une partie dorsale (28) formée par des boucles de fil hydrophile faisant saillie vers le bas à partir de ladite partie basale centrale (27) et définissant une surface inférieure de la première couche (25),
ladite partie fondamentale centrale (27) réunissant les parties supérieure et dorsale (26, 28) tout en fournissant une séparation structurelle intégrée entre elles, caractérisé en outre en ce que
la partie supérieure (26) a le pouvoir de jouer le rôle de mèche à liquides à partir de la surface supérieure (35) dudit coussinet (20),
la partie dorsale (28) a le pouvoir d'absorber les liquides et d'assurer une fonction de réservoir pour assurer la présence minimale de liquide dans la partie supérieure (26) et notamment dans la surface supérieure (35) en contact avec l'incontinent et
ladite partie fondamentale centrale (27) a une construction et un pouvoir de rétention de liquides qui facilitent la dispersion du liquide de la partie supérieure (26) dans la partie dorsale (28),
pour prévenir ainsi le retour du liquide de la partie dorsale (28) à la partie supérieure (26) de sorte que
la surface supérieure (35) du coussinet pour incontinent (20) donne une impression de sécheresse peu après que le liquide y a été excrété.

2. Coussinet selon la revendication 1, caractérisé en ce que ladite partie basale centrale (27) est entièrement en polyester.

3. Coussinet selon la revendication 1, caractérisé en ce que ladite partie basale centrale (27) est en une combinaison de polyester et de coton.

4. Coussinet selon la revendication 1, caractérisé en ce que lesdites parties supérieure et dorsale (26, 28) sont entièrement en polyester.

5. Coussinet selon la revendication 1, caractérisé en ce que ladite partie basale centrale (27) et ladite partie dorsale (28) de ladite monocouche (25) sont en coton.

6. Coussinet selon l'une quelconque des revendications 1 à 5, caractérisé en ce que le coussinet (20) a un poids compris grossièrement dans la plage de 8,5 onces par yard carré à grossièrement 12,5 onces par yard carré (288 à 423 g/m²).

7. Coussinet selon la revendication 4, caractérisé en ce que ledit polyester de ladite partie supérieure (26) est une variété de polyester différente du polyester de ladite partie basale centrale (27) et de ladite partie dorsale (28).

8. Coussinet selon l'une quelconque des revendications 1 à 7, caractérisé en ce que ladite monocouche (25) a un poids compris entre grossièrement 8,5 onces par yard carré et grossièrement 12,5 onces par yard carré (288 et 423 g/m²).

9. Coussinet selon la revendication 5, caractérisé en ce que ladite partie dorsale (28) de ladite monocouche (25) est à 100% en coton.

10. Coussinet selon la revendication 9, caractérisé en ce que ladite monocouche (25), comprenant également ladite partie basale centrale (27), comprend entre 25 et 62% de coton et entre 75 et 38% de polyester.

11. Coussinet selon la revendication 10, caractérisé en ce que ladite monocouche (25) a un poids compris dans la plage de grossièrement 8,5 onces par yard carré à grossièrement 12,5 onces par yard carré (288 à 423 g/m²).

12. Coussinet selon la revendication 1, caractérisé en ce que ladite partie dorsale (28) de ladite monocouche (25) est en un mélange de polyester et de coton.

13. Coussinet selon la revendication 12, caractérisé en ce que ledit mélange de polyester et de coton dans ladite partie dorsale (28) est constitué par un mélange de 50% de polyester et de 50% de coton.

14. Coussinet selon l'une quelconque des revendications 1 à 13, caractérisé en ce que ladite partie supérieure (26) consiste entièrement en boucles non coupées de tissu qui assurent un effet de mèche à partir de la surface externe de ladite partie supérieure (26).

15. Coussinet selon la revendication 14, caractérisé en ce que lesdites boucles non coupées sont des boucles brossées, ce qui améliore encore ledit effet de mèche.

16. Coussinet selon la revendication 1, caractérisé en ce que ladite partie basale centrale (27) est en un mélange de 50% de coton et de 50% de polyester.

17. Coussinet selon l'une quelconque des revendications 1 à 16, caractérisé en ce que ladite partie basale (27) est constituée entièrement par des boucles non coupées et non brossées de tissu.

18. Coussinet selon la revendication 1, caractérisé en ce que ladite partie basale centrale (27) et ladite partie dorsale (28) de ladite monocouche (25) sont en un tissu ou un mélange de tissus sélectionnés dans le groupe constitué par le polyester, le coton, la rayonne et le Nylon.
